# EUROPEAN PATENT APPLICATION

(11) **EP 1 316 606 A1**
(43) Date of publication of application: **04.06.2003**
(21) Application number: 02025677.2
(22) Date of filing: 20.11.2002
(51) Int. Cl.: C12N 15/10, C12Q 1/68

(54) **Method for separation and purification of nucleic acid and unit for separation and purification of nucleic acid**

(30) Priority: 28.11.2001 JP 2001361840
(71) Applicant: FUJI PHOTO FILM CO., LTD., Kanagawa-ken, 250-0193 (JP)
(72) Inventor: Makino, Yoshihiko, Fuji Photo Film Co., Ltd., Asaka-shi, Saitama 351-8585 (JP); Mori, Toshihiro, Fuji Photo Film Co., Ltd., Asaka-shi, Saitama 351-8585 (JP)
(74) Representative: Banzer, Hans-Jörg, Dipl.-Ing.

(57) **Abstract**

An object of the present invention is to provide a method for separation and purification of nucleic acid, which has excellent separating properties, high washing efficiency, and easy processability, which uses mass-producible solid phases with substantially uniform separating capacities, and wherein procedures can be automated, and a unit for separation and purification of nucleic acid suitable for implementing this method. The object of the present invention was attained by a method for separation and purification of nucleic acid comprising the steps of adsorbing nucleic acid onto a solid phase' and desorbing the nucleic acid from the solid phase, wherein the solid phase comprises a base material in which a graft polymer chain having a hydrophilic group is bonded onto the surface; and a unit for separation and purification of nucleic acid which comprises, in a container having at least two openings, a solid phase of a base material in which a graft polymer chain having a hydrophilic group is bonded onto its surface.

## Description

### Technical Field

The present invention relates to the field of molecular biology. More particularly, the present invention relates to a method for separation and purification of nucleic acid, and a unit for separation and purification of nucleic acid by using this method.

### Background Art

Nucleic acid is used in various fields in a variety of forms. For example, the use of nucleic acid in the form of probes, genomic nucleic acids and plasmid nucleic acids are required in the field of recombinant nucleic acid technology.

Nucleic acid is used in various ways in the field of diagnosis. For example, nucleic acid probes are commonly used for detecting and diagnosing human pathogens. Similarly, nucleic acid is used for detecting genetic disorders. Nucleic acid is also used for detecting food contaminants. Further, nucleic acid is commonly used for localizing, identifying and isolating a nucleic acid of interest in various ways including mapping, cloning and recombinant expression.

In many cases, only a very small amount of nucleic acid is available, and procedures for isolation and purification are complicated and time-consuming. These time-consuming and complicated procedures often result in the loss of nucleic acids. In the separation and purification of sample nucleic acid obtained from serum, urine and bacterial culture, there is a risk of contamination and false positive results.

One widely-known purification method is a method of allowing nucleic acids to be adsorbed onto the surface of silicon dioxide, silica-polymer, magnesium silicate and the like in the presence of chaotropic salt, followed by washing, desorption and the like (for example, JP Patent Publication (Examined Application) No. 7-51065). While this method has excellent separating properties, it has drawbacks, for example, industrial mass-production of the adsorption media having uniform capacity is difficult, the handling is inconvenient, and the processing into various forms is difficult, and thus automation of required procedures is also difficult.

### Summary of the Invention

An object of the present invention is to provide a method for separation and purification of nucleic acid, which has excellent separating properties, high washing efficiency and easy processability, and which uses mass-producible solid phases with substantially uniform separating capacities, and wherein procedures can be automated. This method allows nucleic acids in a specimen to be adsorbed onto the surface of a solid phase, and is followed by desorption through washing and the like. Another object of the present invention is to provides a unit for separation and purification of nucleic acid suitable for implementing this method.

The object of the present invention was attained by a method for separation and purification of nucleic acid comprising the steps of adsorbing nucleic acid onto a solid phase and desorbing the nucleic acid from the solid phase, wherein the solid phase comprises a base material in which a graft polymer chain having a hydrophilic group is bonded onto the surface; and a unit for separation and purification of nucleic acid which comprises, in a container having at least two openings, a solid phase of a base material in which a graft polymer chain having a hydrophilic group is bonded onto its surface. In the present invention, the "nucleic acid" may be single-stranded or double-stranded, and may be DNA or RNA. The molecular weight of nucleic acid is not limited.

The solid phase of a base material in which a graft polymer chain is bonded onto the surface, which is used in the present invention, can adsorb nucleic acid at high density, since one of the terminals of the graft polymer chains is strongly bonded onto the surface of the base material, and the graft polymer chain contains a hydrophilic group such as hydroxyl group, that is, a group which is involved in nucleic acid adsorption in the presence of a nucleic acid adsorption buffer, at high density per unit area on the surface of the solid phase. Further, these graft polymer chains having hydrophilic groups are not cross-linked with each other, and they can move freely in a solution and are maintained in a high mobility state. Thus, the adsorptivity between hydrophilic groups and nucleic acids in the graft polymer chains is enhanced, thereby achieving adsorption and desorption of nucleic acid with high efficiency.

### Brief Description of Drawings

Fig. 1 is a conceptual view showing the solid phase in which the graft polymer chain having a hydrophilic group is bonded onto the surface of the base material.
Fig. 2 is a perspective view showing the unit for separation and purification of nucleic acid which comprises the solid phase.
Fig. 3 is a perspective view showing the unit for separation and purification of nucleic acid when the solid phase itself has a three-dimensional structure formed by micro fabrication.

### Embodiments for Carrying out the Invention

The embodiments of the present invention will be described below in detail.

### [A] Base material

The base material used in the present invention can be selected according to the purpose without particular limitations. Usable materials for the base material include inorganic noncrystalline materials such as glass, cement, ceramics and new ceramics, while organic polymeric materials are preferably used from the viewpoint of easy processability.

Examples of the organic polymeric materials include: cellulose tributyrate, cellulose acetate butyrate, cellulose triacetate, and saponification products thereof; polyacetal; polyamide (including aliphatic polyamide and aromatic polyamide such as aramid); polyamide imide; polyalylate; polyimide; polyether imide (PEI); polyether ether ketone (PEEK); polyether sulfone (PES); polyethylene terephthalate (PET); polyethylene naphthalate (PEN); polycarbonate; polysulfone (PSF); polystyrene; polycellulose triacetate and a saponification product thereof; polyphenylene sulfide (PPS); polyphenylene oxide (PPO); polybenzoxazolle; and amorphous polyacrylate (PAR).

Other organic polymeric materials that can be used as base materials include: synthetic resins such as epoxy, acrylic, urethane, phenol, styrene, vinyl, polyester, polyamide, melamine, and formalin resins; and naturally occurring resins such as gelatin, casein, cellulose, and starch.

The base material may be, for example, nonporous plate-like, columnar, granular (beads), or tube-like, as well as a porous plate-like, columnar, granular (beads), tube-like, or the like, such as porous glass, porous ceramics, porous silicon and membrane filter. Alternatively, a coating comprising a material as described above as a raw material may be integrally formed on the surface of the plate-like, columnar, granular (beads) or tube-like base material. Further, the base material may be in the form of a woven fabric, knitted fabric, or nonwoven fabric, which is formed using a long or short fiber comprising the above-mentioned material as a raw material.

The base material itself may be three-dimensional structure formed by micro fabrication. Also, a coating comprising the above-mentioned material as a raw material may be provided on the surface of the micro fabricated three-dimensional structure. The formation of the base material itself into a three-dimensional structure by micro fabrication increases the surface area of the solid phase. This enables nucleic acids to be separated and purified more efficiently. Also, in combination with micro-fluidics for transporting very small amounts of liquid, nucleic acids can be separated and purified from very small amounts of sample in a very small space.

In the present invention, the base material may include an intermediate layer which is provided on the various base materials as mentioned above.

### [B] Surface

In the present invention, when the base material is porous, the "surface of the base material" includes not only the outer surfaces of the base material, but also the inner surfaces of the base material.

### [C] Hydrophilic group

In the present invention, the hydrophilic group in the graft polymer chain bonded onto the surface of the base material refers to a polar group (atomic group) capable of interacting with water. Any group (atomic group) which is involved in nucleic acid adsorption in the presence of the nucleic acid adsorption buffer falls under this category. The hydrophilic group preferably has moderate interaction with water, and examples thereof include hydroxyl, carboxyl, cyano, and oxyethylene groups, with the hydroxyl group being preferred.

### [D] Nucleic acid adsorption buffer

The nucleic acid adsorption buffer used in the present invention is a water-ethanol solution comprising a main agent, a buffer and optionally a surfactant. Examples of usable main agents include compounds which remove water molecules from hydrated molecules in a solution and are known to be a substance capable of destabilizing the three-dimensional structures of proteins in the solution. Examples of such compounds include guanidine hydrochloride, guanidine isothiocyanate, guanidine thiocyanate, sodium perchlorate, and sodium iodide, with guanidine hydrochloride being particularly preferred. Usable buffers include Tris and EDTA, and usable surfactants include Triton-X100. Among these, a solution containing guanidine hydrochloride and Tris is preferably used. Preferred concentration of the buffer is 10 to 100mM, and preferred concentration of the surfactant is 0.1 to 10%.

### [E] Graft polymer chain

In the present invention, the graft polymer chain, which is bonded onto the surface of the base material, is not particularly limited. Any graft polymer chain can be used as long as it has a hydrophilic group in the polymer chain or in the side chain.

A method for introducing such a graft polymer chain onto the surface of the base material is described. The solid phase used in the present invention is characterized in that a graft polymer chain is bonded onto the surface of the base material. The graft polymer chain may be directly bonded onto the surface of the base material. Alternatively, an intermediate layer to which the graft polymer chain can easily bind may be provided on the surface of the base material, and the polymer chain having a reactive functional group at its terminal may be grafted on the intermediate layer. The solid phase used in the present invention includes those placed on the surface of the base material by using a polymer having a graft polymer chain bonded onto a backbone polymeric compound or a polymer having a graft polymer chain bonded onto a backbone polymeric compound and having a crosslinkable functional group introduced therein.

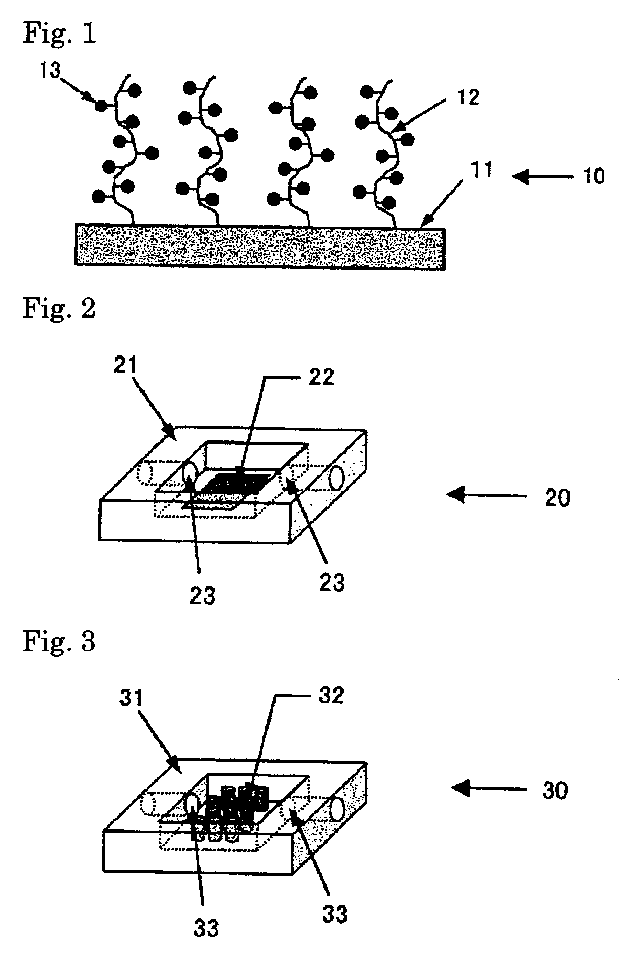

The graft polymer chain used in the present invention has a construction such that a terminus of the polymer is bonded onto the surface of the base material or the surface layer of the base material, and that a grafted portion, in which a hydrophilic group which is involved in nucleic acid adsorption in the presence of a nucleic acid adsorption buffer is present, is not substantially cross-linked. Fig. 1 is a conceptual view showing the solid phase in which the graft polymer chain having a hydrophilic group is bonded onto the surface of the base material. This construction maintains high mobility without restricting the mobility of the polymer portion having a hydrophilic group capable of adsorbing and desorbing nucleic acids and without embedding the solid phase in the strongly cross-linked structure. Accordingly, the adsorptivity of nucleic acid onto a hydrophilic group in the graft polymer chain is enhanced, and the nucleic acid can be adsorbed and desorbed with high efficiency. The molecular weight of such a graft polymer chain is in the range of Mw 500 to 5,000,000, preferably Mw 1,000 to 1,000,000, and more preferably Mw 2,000 to 500,000. If the surface of the base material itself is capable of adsorbing nucleic acids, nucleic acids may be adsorbed onto the surface of the base material.

In Fig. 1, "10" represents a solid phase of the base material in which a graft polymer chain is bonded onto the surface, "11" represents a base material, "12" represents a graft polymer chain, and "13" represents a hydrophilic group.

In the present invention, (1) when the graft polymer chain is directly bonded onto the surface of the base material or is bonded onto the intermediate layer provided on the surface of the base material, it is referred to as a "surface graft", and (2) when the graft polymer chain is introduced into the cross-linked polymer structure, it is referred to as a "graft chain-introduced cross-linked layer".

### (1) Method for producing surface graft

There are two methods for binding the graft polymer chain onto the surface of the base material: one method is carried out by chemical bonding; and another method is carried out by polymerizing compounds having a double bond which is polymerizable with the base material, thereby forming a graft polymer chain.

A method for chemically bonding a base material with a graft polymer chain is first described. In this method, a polymer having a functional group that is reactive with a base material at the terminus or side chain of a polymer is used. This functional group is chemically reacted with a functional group on the surface of the base material, thereby resulting in grafting. This functional group that is reactive with a base material is not particularly limited as long as it can react with a functional group on the surface of the base material, and examples thereof include silane coupling groups such as alkoxysilane, and isocyanate, amino, hydroxyl, carboxyl, sulfonic acid, phosphoric acid, epoxy, allyl, methacryloyl, and acryloyl groups.

Particularly useful polymer compounds that have a reactive functional group at the terminus or side chain include a polymer having a trialkoxysilyl group at its terminus; a polymer having an amino group at its terminus; a polymer having a carboxyl group at its terminus; a polymer having an epoxy group at its terminus; and a polymer having an isocyanate group at its terminus. A polymer used herein is not particularly limited as long as it has a hydrophilic group which is involved in nucleic acid adsorption in the presence of the nucleic acid adsorption buffer. Specific examples thereof include: polyhydroxyethyl acrylate, polyhydroxyethyl methacrylate, and salt thereof; polyvinyl alcohol, polyvinyl pyrrolidone, polyacrylate, polymethacrylate, and salt thereof; and polyoxyethylene. In addition, a polymer of monomers having hydrophilic groups involved in the nucleic acid adsorption in the presence of the nucleic acid adsorption buffer, or a copolymer comprising such monomers, which are used in the following surface graft polymerization, can be advantageously used.

A method for forming a graft polymer chain by polymerizing a compound having a double bond which is polymerizable with the base material is generally referred to as "surface graft polymerization." The surface graft polymerization is a method in which an active species is applied onto the surface of the base material by plasma exposure, photo-irradiation, heating, and the like, and a compound having a polymerizable double bond, which is placed in contact with a base material, is bonded to the base material by polymerization.

Any conventional method of surface graft polymerization described in literature can be used in order to carry out the present invention. For example, a photo-induced graft polymerization method and a plasma-induced graft polymerization method are described as surface graft polymerization methods in "New Polymer Experiment 10," The Society of Polymer Science, Japan, (Ed.), Kyoritsu Shuppan Co., Ltd., 1994, pp. 135. "KYUCHAKU-GIJUTSU BINRAN (Manual for Adsorption Technology)," NTS, Takeuchi (Ed.), 1992. 2 (pp. 203, pp. 695) describes radiation-induced graft polymerization by gamma-ray, electron, and the like. Specific examples of photo-induced graft polymerization usable herein include methods described in JP Patent Publication (Unexamined Application) No. 63-92658, JP Patent Publication (Unexamined Application) No. 10-296895, and JP Patent Publication (Unexamined Application) No. 11-119413. Methods described, for example, in the above-mentioned literatures and Y. Ikeda et al, Macromolecules, Vol. 19, pp. 1804 (1986) can be applied in plasma-induced graft polymerization and radiation-induced graft polymerization.

More specifically, the surface of a polymer such as PET is processed with plasma or electron to generate radicals on the surface. Thereafter, the active surface is allowed to react with a monomer having a hydrophilic group, thereby obtaining a surface layer having the graft polymer chain bonded thereon.

Photo-induced graft polymerization can be carried out by coating a photo-polymerizable composition on the surface of a film base material, bringing an aqueous radical polymerizable compound into contact with the surface and applying light, as described in JP Patent Publication (Unexamined Application) No. 53-17407 (Kansai Paint Co., Ltd.) or JP Patent Publication (Unexamined Application) No. 2000-212313 (Dainippon Ink And Chemicals, Incorporated) as well as the previously mentioned literature.

### (Compound having a polymerizable double bond useful for surface graft polymerization)

In the present invention, the compounds which are useful for forming graft polymer chains bonded onto the base material, are required to have a polymerizable double bond and have a hydrophilic group in its molecule, which is involved in nucleic acid adsorption in the presence of the nucleic acid adsorption buffer. Any polymer, oligomer or monomer compounds having a hydrophilic group can be used as the above-described compounds as long as they have a double bond in the molecule. A particularly useful compound is a monomer having a hydrophilic group. The monomer having a hydrophilic group which is useful in the present invention is a monomer having hydroxyl, carboxyl, cyano, or the like.

In the present invention, specific examples of particularly useful monomers having a hydrophilic group include the following monomers. For example, hydroxyl-containing monomers such as 2-hydroxyethyl acrylate, 2-hydroxyethyl methacrylate, and glycerol monomethacrylate can be particularly preferably used. Carboxyl-containing monomers such as acrylic acid and methacrylic acid or alkali metal salt thereof and amine salt thereof can be preferably used.

### (2) Method for producing graft chain-introduced cross-linked layer

The cross-linked layer having a graft chain introduced therein according to the present invention can be produced by preparing a graft polymer chain by conventional known method for synthesizing a graft polymer, and cross-linking the graft polymer chain. Synthesis of a graft polymer is described in "GURAFUTO JUGO TO SONO OYOU (Graft polymerization and application thereof)," Fumio Ide, 1977, KOBUNSHI KANKOKAI, and "New Polymer Experiment 2, Synthesis and Reaction of Polymers," The Society of Polymer Science, Japan, (Ed.), Kyoritsu Shuppan Co., Ltd. (1995).

The synthesis of a graft polymer is basically classified into: 1) a branched monomer is polymerized from a backbone polymer; 2) a branched polymer is bonded onto a backbone polymer, and 3) a branched polymer is copolymerized with a backbone polymer (macromer technology). A base material having a graft polymer chain bonded onto its surface can be produced by any of these three methods. Among them, 3) "The macromer technology" is particularly excellent from the viewpoint of production suitability and membrane structure control. The synthesis of the graft polymer chain using the macromer technology is described in "New Polymer Experiment 2, Synthesis and Reaction of Polymers," The Society of Polymer Science, Japan, (Ed.), Kyoritsu Shuppan Co., Ltd. (1995). It is also described in detail in "MAKURO MONOMA NO KAGAKU TO KOGYO (Chemistry and Industry of Macromomoer)," Yu Yamashita et al., IPC (1989).

Particularly useful macromers having a hydrophilic group used in the present invention are: macromers which are derived from hydroxyl-containing monomers such as 2-hydroxyethyl acrylate, 2-hydroxyethyl methacrylate, and glycerol monomethacrylate; and macromers which are derived from carboxyl-containing monomers such as acrylic acid and methacrylic acid. The useful molecular weight of these macromers is in the range of 100 to 100,000, preferably in the range of 1,000 to 50,000, and particularly preferably in the range of 1,500 to 20,000. With a molecular weight of 400 or lower, the effect cannot be obtained. With a molecular weight of 100,000 or higher, polymerizability with a copolymerized monomer which forms a main chain is deteriorated.

One method for producing a cross-linked layer having a graft chain introduced therein after the synthesis of these macromers is carried out by copolymerizing the macromer having a hydrophilic group with another monomer having a hydrophilic group to synthesize a graft copolymer, coating the synthesized graft copolymer and a cross-linking agent which reacts with the hydrophilic group of the polymer on the base material, and allowing the reaction to proceed and then cross-linking by heating. Other methods include a method in which a macromer having a hydrophilic group and a graft polymer chain having a photo-crosslinking or polymerizable group are synthesized, and the synthesized products are coated on the base material and allowed to react and crosslink by photo irradiation.

Subsequently, a method for purification of nucleic acid, which uses the solid phase of a base material in which a graft polymer chain having a hydrophilic group is bonded onto its surface, is described. The sample solution containing nucleic acid that can be used in the present invention is not particularly limited, and examples thereof in the field of diagnosis include solutions prepared from body fluids such as whole blood, blood plasma, serum, urine, feces, semen, saliva, or expectoration which are collected as specimens.

These specimen solutions are first processed with a solution containing a reagent which dissolves and destructs cell membranes. This results in dissolution and destruction of a nuclear membrane, and nucleic acid is dispersed in the solution. Reagents which dissolve and destruct cell membranes include Protease K.

A nucleic acid adsorption buffer is added to the solution comprising nucleic acids dispersed therein to bring the nucleic acids into contact with the solid phase. This procedure allows nucleic acids in the sample solution to be adsorbed onto the hydrophilic group of the graft polymer chain of the solid phase, and onto the base material of the solid phase in some cases.

The specimen solution can also be treated by using a solution obtained by adding a reagent for dissolving and destructing cell membranes into the nucleic acid adsorption buffer.

Subsequently, the solid phase having the nucleic acid adsorbed thereon is brought into contact with a washing buffer. The washing buffer has a function of washing away the impurities in the sample solution that were adsorbed onto the solid phase together with the nucleic acids. Accordingly, the washing buffer is required to have a composition which desorbs only impurities from the solid phase and not the nucleic acids. The washing buffer is a solution comprising a main agent, a buffer, and if necessary, a surfactant. The main agent includes an approximately 10 to 90% (preferably about 50 to 90%) aqueous solution of methyl alcohol, ethyl alcohol, butyl alcohol, acetone and the like, and the buffer and surfactant include the buffers and surfactants mentioned above. Among them, a solution containing ethyl alcohol, Tris, and Triton-X100 is preferably used. Preferred concentration of Tris is 10 to 100 mM, and preferred concentration of Triton-X100 is 0.1 to 10%.

The washed solid phase is then brought into contact with a solution capable of desorbing the nucleic acids that have been adsorbed onto the solid phase. Since the resulting solution after contacting with the solid phase will contain the nucleic acids of interest, this is collected and then subjected to subsequent procedures, for example, nucleic acid amplification by Polymerase Chain Reaction (PCR). Usable solutions capable of desorbing the nucleic acid include purified distilled water and TE buffer.

In actual procedure of separation and purification of nucleic acid, it is preferred to use a unit for separation and purification of nucleic acid which has a solid phase in a container (holder) having at least two openings. A material of the container (holder) is not particularly limited as long as it can be equipped with a solid phase and can be provided with at least two openings. Plastic is preferred from the viewpoint of easy producibility.

Fig. 2 is a conceptual view showing the unit for separation and purification of nucleic acid according to the present invention, which has a solid phase in a container having at least two openings. Fig. 3 is a conceptual view showing the unit for purification of nucleic acid according to the present invention when the solid phase itself has a three-dimensional structure formed by micro fabrication. Basically, the unit is sufficient if it has a container for containing the solid phase, and does not allow the solid phase to slip off from the container during the transportation of sample liquids and the like. The sample liquid can be transported using air pressure, centrifugal force, electro-osmotic force, electrophoretic force, or the like.

In Fig. 2, "20" is one embodiment of the unit for separation of nucleic acid according to the present invention, "21" is a holder, "22" is a solid phase according to the present invention, and "23" is an opening in the holder. In this embodiment, holder 21 may be formed independently of solid phase 22. Alternatively, the holder itself may also serve as a base material for forming solid phase 22. In the latter embodiment, a polymer having a hydrophilic group is bonded onto a material for forming a holder.

In Fig. 3, "30" is another embodiment of the unit for separation of nucleic acid according to the present invention, "31" is a holder, "32" is the solid phase according to the present invention in which the base material itself is a three-dimensional structure formed by micro fabrication and "33" is an opening in the holder. In this embodiment, holder 31 may be formed independently of solid phase 32. Alternatively, a part of the holder 31 itself may be a three-dimensional structure formed by micro fabrication, and may also serve as a base material for forming solid phase 32.

A sample solution containing nucleic acid is introduced into one opening of the unit for separation and purification of nucleic acid, the sample solution is transported with the aid of air pressure and the like to be in contact with the solid phase, and then the sample solution is discharged through another opening. Subsequently, the washing buffer is introduced into one opening so that the solution can come into contact with the solid phase, and is then discharged through another opening. A solution capable of desorbing the nucleic acid adsorbed on the solid phase is then introduced into one opening to be in contact with the solid phase, discharged through another opening, and this discharged liquid is collected. Thus, the nucleic acid of interest can be obtained.

Alternatively, the nucleic acid of interest can be obtained by successively immersing the solid phase into a sample solution containing nucleic acid and a nucleic acid adsorption buffer, a nucleic acid washing buffer, and a solution capable of desorbing the nucleic acid adsorbed on the solid phase.

The present invention will be described in more detail with reference to the following examples, but the present invention is not limited thereto.

### Examples

### (a) Production of solid phase (base material having a graft polymer chain bonded onto its surface)

An oxygen glow discharge treatment was carried out under the conditions described below using a biaxially stretched polyethylene terephthalate film (thickness: 188 µ m, A4100, manufactured by Toyobo Co., Ltd.) and a planar magnetron sputtering device (CFS-10-EP70, manufactured by Shibaura Eletec Corporation).

| (Conditions for oxygen glow discharge treatment) | |
|---|---|
| Initial vacuum | 1.2 x 10⁻³Pa |
| Oxygen pressure | 0.9 Pa |
| RF-glow | 1.5 KW |
| Processing time | 60 sec |

Subsequently, the glow-treated film was immersed in an aqueous solution (10 wt%) of 2-hydroxyethyl acrylate which was bubbled with nitrogen, at 70°C for 5 hours. Thereafter, the surface of the immersed film was washed with methanol for 5 hours to obtain a base material having a graft polymer chain of hydroxyethyl acrylate on its surface (Solid Phase 1).

### (b) Production of a unit for separation and purification of nucleic acid

A unit for separation and purification of nucleic acid having two openings and a container for containing Solid Phase 1 produced in (a) above (base material area: 1.0 cm²), was prepared with High Impact Polystyrene.

### (c) Preparation of nucleic acid adsorption buffer and washing buffer

A nucleic acid adsorption buffer and a washing buffer having the following formulations were prepared.

| (Nucleic acid adsorption buffer) | |
|---|---|
| Guanidine hydrochloride (Life Technologies Inc) | 382 g |
| Tris (Life Technologies Inc) | 12.1 g |
| Triton-X100 (ICN) | 10 g |
| Ethanol | 300 ml |
| Distilled water | 700 ml |

| (Washing buffer) | |
|---|---|
| 100 mM Tris-HCl | 70% ethanol |

### (d) Separation and purification of nucleic acid

100 µ L of human whole blood was collected using a vacuum blood collecting tube, and 100 µ L of the nucleic acid adsorption buffer with the formulation as shown in (c) above and 10 µ L of Protease K were added thereto. The mixture was then incubated at 60°C for 10 minutes. After incubation, 100 µ L of ethanol was added and stirred therein. After stirring, the sample solution which was treated as mentioned above was introduced into one opening of the unit for separation and purification of nucleic acid produced in (b), and the sample solution was brought into contact with the surface of Solid Phase 1. Further, the liquid was sucted into the unit for separation and purification of nucleic acid using a syringe which was connected to another opening of the unit for separation and purification of nucleic acid, followed by discharging.

Immediately after discharging, 0.5 mL of washing buffer was introduced into one opening of the unit for separation and purification of nucleic acid. The liquid was sucted into the unit for separation and purification of nucleic acid using a syringe which was connected to another opening of the unit for separation and purification of nucleic acid, followed by discharging. The inside of the unit for nucleic acid purification and the surface of Solid Phase 1 were then washed.

After washing the inside of the unit for purification of nucleic acid and the surface of Solid Phase 1, 100 µL of purified distilled water was introduced into one opening of the unit for separation and purification of nucleic acid and brought into contact with the surface of Solid Phase 1. Thereafter, the liquid was sucted into the unit for separation and purification of nucleic acid using a syringe which was connected to another opening of the unit for separation and purification of nucleic acid, followed by discharging. The discharged liquid was then collected.

### (e) Confirmation of the collected amount, purity, and PCR amplification of nucleic acid

### (Confirmation of the collected amount and purity of nucleic acid)

The absorbance of the discharged liquid collected in (d) above was measured, and the collected amount and purity of the nucleic acid were quantified. The collected amount was quantified by the absorbance at the wavelength of 260 nm, and the purity of nucleic acid was determined based on the absorbance ratio at 260/280 nm (A260/A280: the purity is determined good if this ratio is 1.8 or higher). As a result, the collected amount was 5.4 µg, and the purity of A260/A280 was 1.902.

### (Confirmation of PCR amplification)

A nucleic acid fragment was amplified by PCR using a reaction solution having the formulation below. PCR was carried out by repeating 30 cycles of denaturing at 94°C for 30 seconds, annealing at 65°C for 30 seconds, and polymerase elongation at 72°C for 1 minute.

| <Formulation of reaction solution> | |
|---|---|
| Purified water | 36.5 µL |
| 10 x PCR buffer | 5 µL |
| 2.5 mM dNTP | 4 µL |
| Tap EP (Nippon Gene Co., Ltd.) | 0.5 µL |
| 20 µ M primer | 2 µL |
| Discharged solution collected in (d) | 2 µL |

A set of synthesized oligonucleotide primers (primer 1 and primer 2), which have a nucleotide sequence designed to specifically recognize exon 6 of p53 gene, was used.

### <Nucleotide sequences of primers>

Primer 1: 5'-GCGCTGCTCAGATAGCGATG-3'

Primer 2: 5’-GGAGGGCCACTGACAACCA-3

Gel electrophoresis was performed by conventional techniques using a solution after PCR amplification. As a result, a single band of PCR product was observed at the expected position of 275 bp.

As is apparent from these results, nucleic acid can be separated and purified from the sample solution with high purity using Solid Phase 1 (base material having graft polymer chains of hydroxyethyl acrylate on its surface) and the unit for separation and purification of nucleic acid which has Solid Phase 1, and PCR can be carried out using the resulting nucleic acid

### Industrially Applicability

High purity nucleic acid can be separated from sample solutions containing nucleic acids by the method for separation and purification of nucleic acid according to the present invention, which has excellent separating properties, high washing efficiency, and easy processability, and which uses mass-producible solid phases with substantially uniform separating capacities, and wherein procedures can be automated. Further, the use of the unit for separation and purification of nucleic acid according to the present invention facilitates this method.

## Claims

1. A method for separation and purification of nucleic acid comprising the steps of adsorbing nucleic acid onto a solid phase and desorbing the nucleic acid from the solid phase, wherein the solid phase comprises a base material in which a graft polymer chain having a hydrophilic group is bonded onto the surface.

2. The method for separation and purification of nucleic acid according to claim 1, wherein the hydrophilic group is a hydroxyl group.

3. A unit for separation and purification of nucleic acid which comprises, in a container having at least two openings, a solid phase of a base material in which a graft polymer chain having a hydrophilic group is bonded onto its surface.
